# EUROPEAN PATENT APPLICATION

(11) **EP 3 000 869 A1**
(43) Date of publication of application: **30.03.2016**
(21) Application number: 14800833.7
(22) Date of filing: 09.05.2014
(51) Int. Cl.: C12N 1/00, C12M 1/00, C12M 3/00, C12N 5/07

(54) **CELL CULTURING METHOD, PARTICULATE CULTURE CARRIER, AND PARTICLE-ENCOMPASSING CELL AGGREGATE**

(30) Priority: 23.05.2013 JP 2013109154
(71) Applicant: Hitachi High-Technologies Corporation, Tokyo 105-8717 (JP)
(72) Inventor: HISADA Akiko, Tokyo 100-8280 (JP); SONODA Hiroshi, Tokyo 100-8280 (JP)
(74) Representative: MERH-IP Matias Erny Reichl Hoffmann Patentanwälte PartG mbB
(86) International application number: PCT/JP2014/062495
(87) International publication number: WO 2014/188888

(57) **Abstract**

Provided is a culturing method capable of causing a three-dimensional aggregate in which cell function is similar to in vivo function to adhere to a culturing vessel while improving permeability of nutrients, oxygen, drugs and reagents from the culture medium and maintaining cell function. By sowing isolated cells in a culturing vessel, and after the cells have settled on the culturing surface, administering microscaffold particles that are cell-adhesive and substance-permeable onto the cells, it is possible to cause the cells to adhere to the culturing vessel while forming cell aggregates that encompass the particles. For example, cell aggregates encompassing the particles are formed by administering gelatin particles in which the size of the culture carrier to be encompassed in the cell aggregate is at least 1/5 of the cell diameter at a cell number:particle number of at least 80:1 with respect to the number of cultured cells per unit culturing area.

## Description

### Technical Field

The present invention relates to a particulate culture carrier, and specifically relates to a cell culturing technique which uses a particle-encompassing cell aggregate in a culture to adhere cells to a culturing vessel.

### Background Art

Patent Literature 1 is background art in the technical field. Patent Literature 1 describes a microsphere-containing cell aggregate produced from hydrogel microspheres and cells and a method for the production thereof. Further, Patent Literature 2 describes a gellable cell culture substrate in which gelatin and boron are used as essential raw materials, and Patent Literature 2 describes that the optimal particle size is a diameter of 5 to 100 µm, and more preferably is a diameter of 10 to 70 µm.

Patent Literature 3 which is another background art describes that gelatin microspheres having a desired size of (10 to 50 µm, etc.) are arranged in passage surfaces partitioned by microsize pillars to create a three-dimensional culture of cells.

Patent Literature 4 which is another background art describes a method for culturing a culture substrate including a fine particle-containing polymer gel particle with a cell in an aggregate of the fine particle-containing polymer gel particles and recovering a three-dimensional tissue. Further, Patent Literature 5 describes an example for supporting and culturing a cell on the surface of a microcarrier having a particle size from 0.1 to 500 µm including a gelatin hydrogel.

### Citation List

### Patent Literature

Patent Literature 1: WO2011-059112
Patent Literature 2: Japanese Unexamined Patent Application Publication No. 2011-130720
Patent Literature 3: US2011/0256574
Patent Literature 4: Japanese Unexamined Patent Application Publication No. 2005-027532
Patent Literature 5: US2005/0054101

### Summary of the Invention

### Technical Problem

The development of a culturing technique for maintaining the function of a cell to be cultured in a state similar to in vivo for a long period of time has been sought in research for developing new drugs and the industrialization of regenerative medicine-related technology, etc.

The fundamental unit of the body is the cell. Moreover, the smallest constituent unit having biological function is a cell aggregate which is formed by cell-cell adhesion as the basic structure. Therefore, a cell aggregate preparation which aggregates cells in vitro has been an important elemental technique in the biomedical research of cells in vitro and the industrial application of cells. For example, it is known that the biological function of a cell aggregate is high and the sensitivity to drugs becomes high even in drug screenings which use cells compared to free single cells, and it is considered that research and toxicity evaluations of drug effects are possible using a cell aggregate similar to in vivo.

Under these circumstances, research to efficiently produce cell aggregates and increase the biological function has been performed. However, decreases in the efficiency of the supply of nutrients and oxygen to the inside of the cell aggregate and the removal of the waste products from the inner cell are of particular concern as problems in the technical field. As the result of the supply and the excretion efficiency decreasing, the cell function decreases and it becomes difficult to use the cell aggregate. However, when analyzing the reaction of cells for the purpose of drug screening, etc., the decrease in the permeability of a target drug and analytical reagents into the interior of the cell aggregate becomes a problem.

In order to solve these problems, the technology which improves the substance-permeability between the interior and the exterior of the aggregate without impairing cell-cell adhesion within the cell aggregate is essential. The aforementioned Patent Literature 1 reports a method to encompass a polymeric hydrogel in a cell aggregate which improves the substance-permeability to the interior of the cell aggregate as a three-dimensional aggregate of the cells. The hydrogel particle has permeability of nutrients and oxygen, thus, it is considered that a three-dimensional aggregate with the particle dispersed therein results in improvements in the problems of the insufficiency of nutrients and oxygen in the inner cell and the accumulation of waste products. Patent Literature 1 reports a method for producing an aggregate having a diameter close to 1 mm which incorporated a microsphere by mixing proliferating cells and hydrogel microspheres and sowing in a non-adhesive culturing vessel.

However, when using a cell aggregate in drug screening, etc., and in vitro cell testing, it is necessary to maintain the cell aggregate in the vessel during the treatment of the test solution, washing, and the like. However, in a culture of cell aggregates formed in an adhesive vessel, a method to encompass a particle having substance-permeability in the interior of the aggregate in a state in which the aggregate is adhered inside the culturing vessel has not been reported until now.

The object of the present invention, taking the above viewpoint into account, is to provide a cell culturing method, a particulate culture carrier, and a particulate-encompassing cell aggregate for adhering a three-dimensional aggregate having cellular function similar to in vivo function in a culturing vessel, while improving the permeability of nutrients, oxygen, drugs, reagents, and the like from the culture medium. Solution to Problem

In order to achieve the aforementioned object, the present invention provides a cell culturing method for forming a cell aggregate for encompassing a particle by sowing isolated cells in a culturing vessel and administering particles that are cell-adhesive and substance-permeable.

Further, to achieve the aforementioned object, the present invention provides a particulate culture carrier including particles that are cell-adhesive and substance-permeable and which are to be encompassed in the cell aggregate produced by administering the particles onto the sown cells in the culturing vessel.

Furthermore, to achieve the aforementioned object, the present invention provides a particulate-encompassing cell aggregate formed by sowing cells in a culturing vessel and administering particles that are cell-adhesive and substance-permeable onto the cells.

### Advantageous Effects of the Invention

According to the present invention, a culturing method which maintains a cultured cell having a function similar to in vivo function for a long period of time and which can be used in in vitro cell testing can be provided in a culture of cells.

### Brief Description of the Drawings

Fig. 1A is a drawing illustrating an example in which particles having different diameters were provided and the metabolic enzyme cytochrome P450 (molecular species CYP3A) activity of a rat hepatocyte aggregate cultured for five days were measured according to a first embodiment.
Fig. 1B is a drawing illustrating an example which the CYP3A activity of a rat hepatocyte aggregate cultured for seven days in the same manner was measured according to the first embodiment.
Fig. 1C is a drawing illustrating an example which the CYP3A activity of a rat hepatocyte aggregate cultured for eleven days in the same manner was measured according to the first embodiment.
Fig. 1D is a drawing showing the culture conditions of the measurement examples in Fig. 1A to Fig. 1C according to the first embodiment.
Fig. 2 is a drawing illustrating an example of the change of the cell aggregate formation over time after the particles having different diameters were encompassed in rate hepatocytes and cultured for 11 days according to the first embodiment.
Fig. 3 is a drawing illustrating an example of the microscope observation image of the gelatin particle suspensions having different diameters and swollen in a culture medium according to the first embodiment.
Fig. 4 is a drawing illustrating an example of the cross section image of the rat hepatocyte aggregate which encompassed particles having different particle sizes according to a second embodiment.
Fig. 5A is a drawing illustrating an example in which the basal activity of CYP3A was measured according to a third embodiment.
Fig. 5B is a drawing illustrating an example in which the drug-induced activity of CYP3A was measured according to the third embodiment.
Fig. 6A is a drawing illustrating an example of the change over time by a microscopic observation when rat hepatocytes were sown in a low-adhesion culturing vessel according to the third embodiment.
Fig. 6B is a drawing illustrating an example of the change of the major axis of a cell over time measured from microscope images when rat hepatocytes were sown in low-adhesion culturing vessel according to the third embodiment.
Fig. 7 is a drawing illustrating an example in which rat hepatocytes were cultured and the activity of mitochondrial dehydrogenase was measured by a WST-1 assay according to a fourth embodiment.
Fig. 8 is a drawing illustrating an example of the change of the cell aggregate formation over time when particles were administered at different ratios relative to the cell number and cultured for five days according to a fifth embodiment.
Fig. 9 is a drawing illustrating an example of the uptake of a fluorescent reagent and the excretion to the bile canaliculus when particles were administered at different ratios relative to the cell number and cultured for five days according to the fifth embodiment.
Fig. 10A is a drawing illustrating an example of the multi-well plate for the cell culture used in each example according to a sixth embodiment.
Fig. 10B is a partial enlarged view of the multi-well plate for the cell culture used in each embodiment according to the sixth embodiment.
Fig. 11 is a drawing illustrating an example of a scanning electron microscope photograph of the culturing surface in which the aggregation region of a projection is molded in the center part of a hole according to the sixth embodiment.
Fig. 12 is a drawing illustrating an example wherein the cell aggregate encompassing the particle is molded in the aggregation region of the projection molded in the center part of the hole according to the sixth embodiment. Description of Embodiments

The present invention is the invention of a cell culturing method for forming a cell aggregate encompassing a particle by sowing isolated cells in a culturing vessel and administering particles that are cell-adhesive and substance-permeable, a particulate culture carrier, and a particulate-encompassing cell aggregate. Optimally, the particles that are cell-adhesive and substance-permeable are microscaffold (micro scaffold) particles which are cell-scaffold particles, and the administration of the particle is performed after the sown cells settle on the culturing surface. More specifically, the adhesion of the cells to the culturing vessel, the adhesion of the cells and the particles, etc., can be ensured by performing the administration of the particle from the time after sowing the isolated cells on the culturing vessel and the cells begin adhering to the culturing surface until the cells move to adhere the adjacent cells to each other and the formation of the cell aggregate begins.

The cell culturing method which uses the microscaffold particles which is a preferred embodiment of the present invention is a culturing method which uses a microparticle obtained by forming chemical crosslinks between the gelatin molecules by a heat treatment, and the like. It is possible to use a chemical crosslinking agent such as glutaraldehyde as the method for producing chemical crosslinks.

The concentration of the gelatin aqueous solution at the time the gelatin particles are produced is desirably a 10% weight concentration, but it is not limited thereto. The procedure for obtaining the microparticle by preparing an emulsion by stirring a gelatin aqueous solution together with oil such as olive oil, cooling the emulsion, degreasing with cold acetone, and the like, and dehydrating may be provided as one method for producing the gelatin particles. It is possible to know the particle size by sieving at this stage. The molecules are crosslinked by heat treating the dried particles.

As an example of another method for preparing the particle, a microreactor (for example, Micro Process Server, manufactured by Hitachi Plant Technologies, Ltd.) is used to uniformly emulsify the gelatin aqueous solution with the olive oil, so that it is possible to obtain a particle wherein the size is controlled.

If the microscaffold particles possess the two properties of cell-adhesiveness of the particle surface and water soluble substance-permeability within the particle, the particle manufactured from any material may be used. In order to have cell-adhesiveness, the surface may be manufactured with a cell adhesion substance or a substance to which the cells adhere. An antibody bound to, for example, collagen, fibronectin, laminin, glycosaminoglycan, a protein on the cell membrane surface, and a sugar chain, a sugar chain bound to the cell membrane surface, or poly-L-lysine, and the like may be provided as the cell adhesion substance, but it is not limited thereto.

In order to have water soluble substance-permeability, a hydrogel containing a large amount of water, or a porous ceramic, a porous metal, and the like may be provided, but it is not limited thereto. As the hydrogel, polyacrylate, polyacrylamide, polyvinylpyrrolidone, polyvinyl alcohol, alginic acid, starch, polylactic acid, agarose, pectin, cellulose, glycosaminoglycan, collagen, gelatin, fibronectin, vitronectin, laminin, proteoglycan, and the like may be provided, and as the porous ceramic, hydroxyapatite may be provided, but the hydrogel and the porous ceramic are not limited thereto.

The particulate-encompassing cell aggregate such as the microscaffold particles of the present invention is a cell aggregate formed by sowing cells showing adhesion to the culturing surface onto a culturing vessel having an adhesion region on the culturing surface, and administering the aforementioned particle after the cells settled on the vessel. The cell aggregate encompassing the particle is formed by the cells adhering to the gelatin which is the raw material in a state in which the cells are adhered to the culturing surface. It is possible to obtain a cell aggregate in which the cell aggregate does not ablate from the culturing equipment material, and without impairing the cell-cell adhesion within the aggregate by selecting the particle administration period, the particle size, and the number of administrations. Specifically, there are cases when the cell functions to be maintained are different depending on the object of use of the particulate-encompassing cell aggregate, thus, it is desirable to adjust the optimal particle administration period, the particle size, and the number of administrations in accordance with the object.

Therefore, the present invention which encompasses a particle in a state in which a cell aggregate is adhered to a culturing vessel is different from the culturing method for adhering cells to the surface of a conventional microcarrier to obtain a useful substance by a suspension culture and the technology for forming a cell aggregate for encompassing a culture carrier with a non-adhesive vessel.

It is possible for the present invention which encompasses a particle in a state in which a cell aggregate is adhered to a culturing vessel to perform drug treatment and the activity measurement of cells by the same operations as the testing which uses a conventional single layer cultured cell. Therefore, the particulate-encompassing cell aggregate which maintains its activity can be easily used in drug screening in research for developing new drugs and cell testing such as investigations of the differentiation inducing conditions in regenerative medicine. Drugs and reagents easily permeate to the inner cell via the substance-permeability microscaffold particles, thus, the problem of the permeability of drugs and reagents which may occur in a cell aggregate which does not encompass the particle can be solved.

Below, each embodiment of the present invention will be explained using drawings.

### First Embodiment

The first embodiment is an example relating to a particulate culture carrier, a particulate-encompassing cell aggregate, and a cell culturing method for culturing hepatocytes.

The hepatocytes used in the embodiment are non-proliferating mature hepatocytes, thus, the functions easily deteriorate during the culturing period. Therefore, the time period in which hepatocytes can be supplied for drug testing, etc., is generally, in the range from the beginning of culturing to one week. The embodiment describes examples in which the period in which the measurement of metabolic enzyme cytochrome P450 (CYP3A) activity is possible was extended to more than 10 days by administering and culturing gelatin particles in rat hepatocytes.

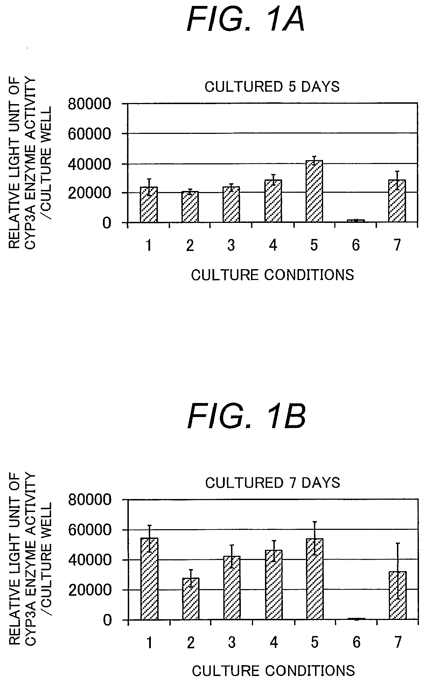

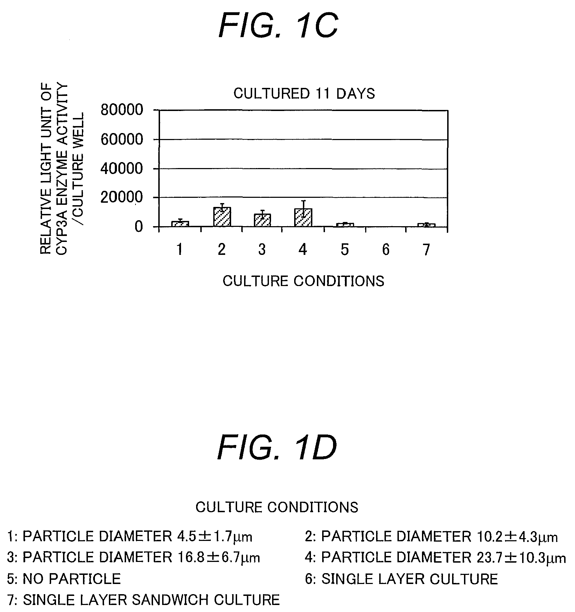

Fig. 1A, Fig. 1B, and Fig. 1C are examples which show that four types of gelatin particles having different diameters were administered to the rat hepatocytes, the enzyme activity was measured over time, and the effect of the enzyme activity maintenance is different due to the particle sizes in the embodiments. Note that, Culture conditions 1 to 7 indicated in these drawings are as indicated in 1 to 7 of Fig. 1D.

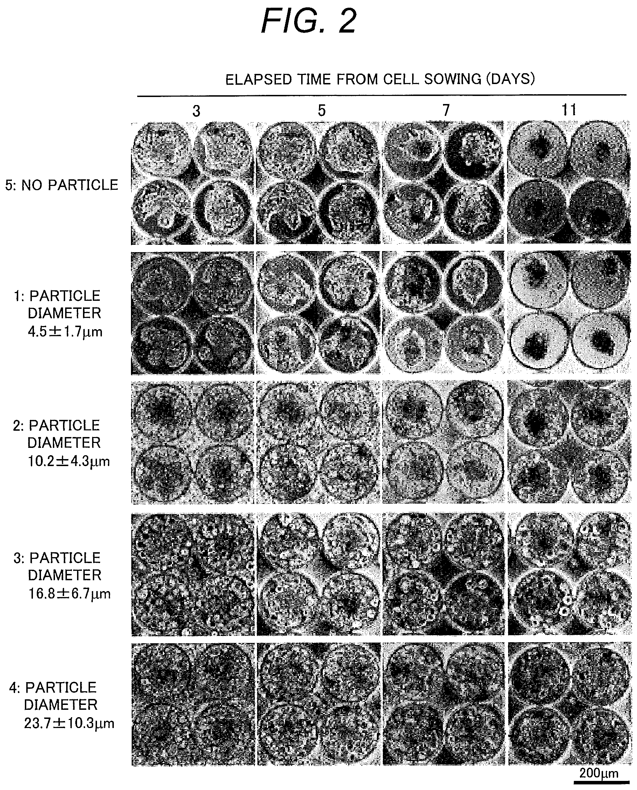

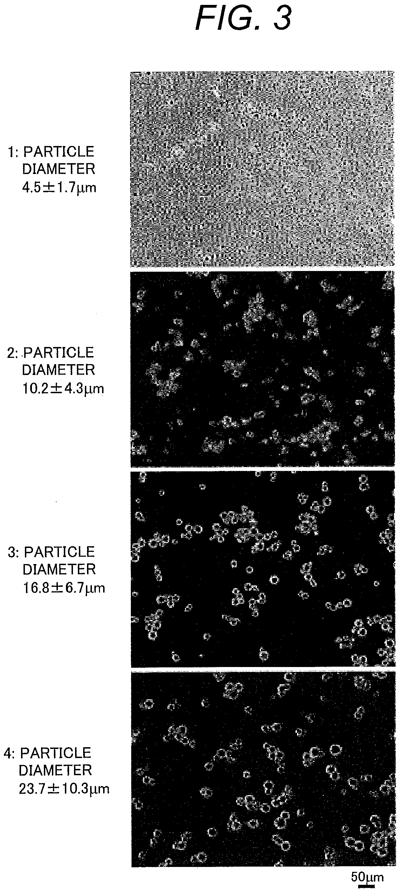

Fig. 2 is an example of the microscope observation images of a sample used in the enzyme activity measurements of Fig. 1A, Fig. 1B, and Fig. 1C and illustrates the change of the cell aggregate formation over time when particles having different diameters were administered to the rat hepatocytes under Conditions 1 to 5 and cultured for 11 days. Fig. 3 shows an example of the microscope observation image of the gelatin particle suspensions having different diameters suspended in the culture medium.

The rat hepatocytes of the embodiments were prepared from Fisher 344 rats or SD rats, male, five to eight weeks of age (Charles River Laboratories Japan) using the two-step collagenase perfusion method. The isolated hepatocytes were suspended in Williams' E culture medium (8.6 nm insulin (Sigma-Aldrich), 255 nm Dexamethasone (Nacalai Tesque), and 50 ng/ml EGF, 5 KIV/ml Aprotinin were added) containing 10% fetal bovine serum, and the cells were sown at a cell concentration of 5×10⁵ cells/ml and at a density of 1×10⁵ cells/cm² per culture area of the culturing vessel. The culture plate was placed in a 37-degree, 5% carbon dioxide gas concentration incubator, and the cells were cultured.

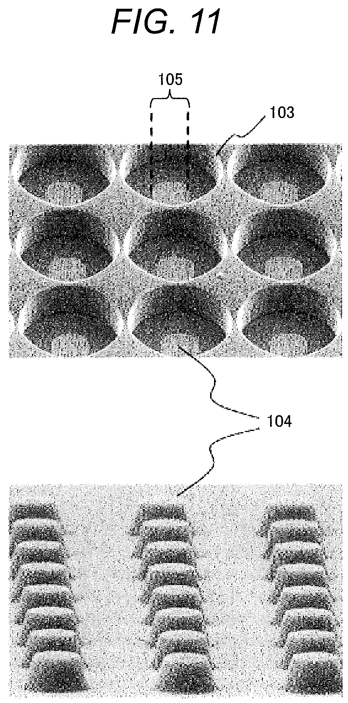

The culturing vessel used in the embodiments, as explained hereinafter using Fig. 11, had numerous hole structures having a 200 µm diameter on the culturing surface, and a 96-well nanopillar culture plate was used in which microprojections having a diameter of 2 µm and a height of 1 µm were processed in the center portion of each hole bottom surface. Refer to, for example, Japanese Unexamined Patent Application Publication No. 2011-004612 and WO2010/079602 by the present inventors. The former describes a culture sheet in which a nanopillar which can control the adhesion and migration of cells was formed. The latter describes the conditions in which hepatocytes easily form a cell aggregate on a nanopillar sheet, and describes that the function of a three-dimensional aggregate is similar to that in vivo by a two-dimensional culturing method.

The material of this culture plate was polystyrene, and the culturing surface having holes and microprojections were subjected to a hydrophilization treatment by surface modification, and had a cell-adhesion property. Specifically, the nanopillar portion which is a microprojection has an effect which captures cells. However, the single layer culture used a BioCoat (BD) 96-well culture plate coated with Type I collagen in the culturing vessel.

The gelatin particles manufactured by the method which is partially modified from the following Non-Patent Literature 1 were used as the particles for administering to cells.

### <Non-Patent Literature 1>

In vitro Proliferation and chondrogenic differentiation of rat bone marrow stem cells cultured with gelatin hydrogel microspheres for TGF-β1 release. Ogawa T. et al., Journal of Biomaterials Science, 21, 609-621 (2010)

A gelatin aqueous solution was manufactured by weighing a dry gelatin powder (isoelectric point: 5), adding purified water to make a 10% weight concentration, and swelling and dissolving at 37 degrees while stirring. Olive oil was heated while stirring in a 40 degrees water bath, the aforementioned gelatin solution was added therein, and emulsified by stirring with a homogenizer (manufactured by Polytron Corporation). The emulsion was cooled while stirring to gelatinize the gelatin. After adding cold acetone while further stirring, the particles were recovered by centrifugation. The particles were washed in cold acetone while passing through a sieve, the particles which passed through the sieve were recovered, and dry gelatin particles were obtained by vacuum drying. Dehydrothermal crosslinking was performed using a vacuum oven to heat under a reduced pressure at 140 degrees for 48 hours. The particles were sterilized with ethylene oxide gas and preserved in the state of sterile dry particles.

The mean value and the standard deviation of the particle size of the gelatin particles were determined by measuring the size under a microscope after the thermal crosslinked particles were swollen in water. The diameters of 4.5±1.7 µm, 10.2±4.3 µm, 16.8±6.7 µm, and 23.7±10.3 µm shown in Fig. 1D were used in the embodiments as the four types of gelatin particles having different sizes.

When administering the particles to cells, 10 mg of dry gelatin particles were dispersed in 50 µl of ethanol, suspended by adding serum-free Williams' E culture medium, and made to a final concentration of ethanol of no more than 1%. The particles were swollen by placing in the culture medium for one hour while stirring. Fig. 3 shows microscope images of the four types of gelatin particles swollen by the culture medium.

The particle concentration of the particle suspension was prepared to 3 mg/ml by dry particle weight. 24 hours after cell sowing, the particles were administered to the cultured cell by replacing the culture medium in which the cells were cultured from Williams' E culture medium containing 10% fetal bovine serum to the serum-free Williams' E culture medium in which the particles were suspended. 48 hours after cell sowing, the Matrigel (BD) was replaced, and subsequently, the serum-free Williams' E culture medium was replaced every 24 hours and the culturing was continued.

The single layer culture which is the conventional method was performed as a comparative experiment. The isolated hepatocytes were suspended at a cell concentration of 5×10⁵ cells/ml, and sown onto the above mentioned culture plate at a density of 1×10⁵ cells/cm² per culture area. The culture plate was placed into a 37-degree, 5% carbon dioxide gas concentration incubator and the cells were cultured. The single layer culture was replaced with the serum-free Williams' E culture medium 24 hours after cell sowing, and subsequently, the serum-free Williams' E culture medium was replaced every 24 hours and the culturing was continued. The single layer sandwich culture replaced the Matrigel (BD) with serum-free Williams' E culture medium containing 0.25 mg/ml total protein 24 hours after cell sowing, and subsequently, the serum-free Williams' E culture medium was replaced every 24 hours and the culturing was continued.

The activity of metabolic enzyme cytochrome P450 (molecular species CYP3A) of hepatocytes was measured using P450-Glo™ Assay (Luciferin-IPA, Promega) five days, seven days, and 11 days from sowing. The procedures for measurement are as follows.

The culture medium in which the hepatocytes were cultured was removed and replaced with 60 µl of Luciferin-IPA solution (final concentration 3 µm) diluted to 1/1000 with the culture medium, and reacted in a 37-degree, 5% carbon dioxide gas concentration incubator for one hour. 50 µl of culture supernatant per well was transferred to a white 96-well plate, 50 µl of luciferin detection reagent (Promega) was added, and was reacted under shading for 20 minutes at room temperature. After reacting, the white plate was placed in a plate reader (SH-8000 Lab, CORONA ELECTRIC Co., Ltd.), and the luminescence was measured at a gate time of one second.

Fig. 1A, Fig. 1B, and Fig. 1C show the 8-well mean value and the standard deviation determined for the culture conditions. In cells cultured for five days, the activity of the cells to which the particles were not administered was high, and the effect of the particles could not be observed in the initial stage of culture. In cells cultured for seven days, the activity of the cells of the aggregate to which the particles having a diameter of 4.5±1.7 µm were administered and the activity of the cells of the aggregate to which the particles were not administered was high.

With respect thereto, in cells cultured for 11 days, the activity of the cells of the aggregate to which the particles having a diameter of 10.2±4.3 µm, 16.8±6.7 µm, and 23.7±10.3 µm were administered was high compared to the other conditions, the activity was low in the cases with particles having the diameter of 4.5±1.7 µm, no particle, and a single layer sandwich culture, and the activity was at the detection limit or less in the most common single layer culture.

Fig. 2 shows microscope photographs in which the cultured hepatocytes were observed with a phase contrast inverted microscope and which were taken of the cell aggregates of the any wells in the vicinity of the center of the culture plate. In the particle administration conditions of the embodiments, the adhesion among the cells in which the particles having a diameter of 4.5±1.7 µm were administered and cultured for three days and five days was promoted more than in cells to which the particles were not administered, and an aggregation trend was observed. The formation of the aggregate cultured for seven days or 11 days was similar to cells to which the particles were not administered, and the aggregates adhered to the region in which the microprojections of the center portion of the hole of the culture plate were aggregated.

Cells to which the particles having a diameter of 10.2±4.3 µm were administered formed aggregates in a state which covered the particles, and the aggregates adhered to the region in which the microprojections of the center portion of the hole of the culture plate were aggregated. Larger aggregates formed than in cultures without the particles, thus, it is considered that the particles are taken up into the cell aggregate.

Cells to which the particles having a diameter of 16.8±6.7 µm and 23.7±10.3 µm were administered were mixed with the particles to form a state in which the holes of the culture plate were filled, and the tendency that the cells cultured for 11 days aggregate with the particles were observed.

If the diameter of the hepatocytes is deemed to be in the range of approximately 15 to 30 µm, the particle size of the particles having a diameter of 4.5±1.7 µm is in the range of approximately 1/10 to 1/5 of the cell, and thus, does not largely influence the function and the shape of cell aggregate. The size of the 11 day aggregate is not remarkably significant compared to those without the particle, thus, the amount of particles administered was 3 mg/ml by dry particle weight for any of the particles, but it is considered that the total amount of the particles take up into the aggregate was small for the particles having a diameter of 4.5±1.7 µm.

With respect thereto, the particles having a diameter of 10.2±4.3 µm in which the particle size is no less than 1.5 of the cell formed an aggregate which encompasses the particle. Further, particles having the diameters of 16.8±6.7 µm and 23.7±10.3 µm formed substantially planar, irregularly shaped aggregates. If the diameter of the hepatocytes is deemed to be in the range of approximately 15 to 30 µm, the particles having a diameter of 23.7±10.3 µm were considered to be in the range of the same size to twice as large as the cell.

As previously stated, the embodiments indicate the effect which measurably maintains the activity of the metabolic enzyme cytochrome P450 (molecular species CYP3A) for no less than 10 days by administering these three types of particles, thus, it is understood that there is an effect for maintaining the metabolic enzyme cytochrome P450 activity of the hepatocytes by forming a cell aggregate encompassing the particle 1.5 times larger than the size of the cell.

### Second Embodiment

The second embodiment describes the examples in the case in which the particles which were encompassed in the cell aggregate were only distributed in the interior of the aggregate due to the particle size when the gelatin particles having different diameters were administered to the rat hepatocytes and cultured, and in the cases not only when distributed in the interior of the aggregate but when the portion in is contact with culture medium.

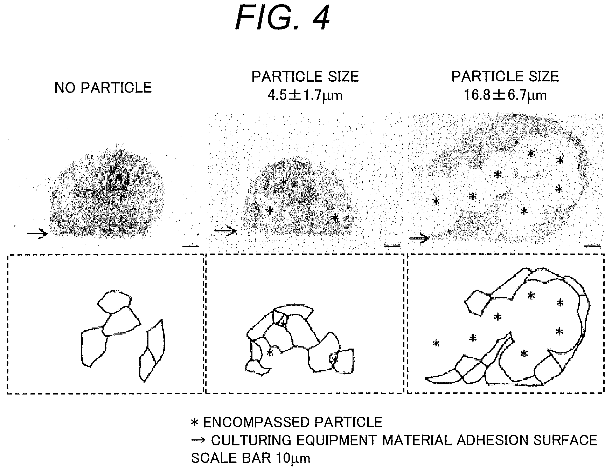

Fig. 4 is an example of microscope photographs showing the cross-section of the gelatin particulate-encompassing cell aggregate in the second embodiment. The schematic illustration of the lower side of the photograph indicates the outline of the cell which can be distinguished at the upper part of the photograph with a line, and indicates the location of the encompassed particles with an arrow.

The culturing vessel described in the aforementioned Japanese Unexamined Patent Application Publication No. 2011-004612 has numerous hole structures having a 200 µm diameter on the culturing surface, and a nanopillar culture sheet in which microprojections having a diameter of 2 µm and a height of 1 µm were processed in the center portion of each hole bottom surface was used by adhering to the well bottom surface of a 2-well chamber slide. The material of the culture sheet is polystyrene, and was subjected to a hydrophilization treatment by surface modification, and has cell-adhesion. Specifically, the nanopillar portion which is a microprojection has the effect for capturing cells.

The isolated hepatocytes were suspended in Williams' E culture medium containing 10% fetal bovine serum at a cell concentration of 5×10⁵ cells/ml, and sown at a density of 1×10⁵ cells/cm² per culture area of the culturing vessel. The chamber slide was placed in a 37-degree, 5% carbon dioxide gas concentration incubator and the cells were cultured.

When administering the particles to cells, 10 mg of dry gelatin particles were suspended in the Williams' E culture medium containing 1 ml of 10% fetal bovine serum, and the particles were swollen with the culture medium. The particle concentration of the suspension of the particles having a diameter of 16.8±6.7 µm was counted by a cell counter (TC10 system, BioRad) and the particle number to be administered was adjusted to be 1/16 of the cell number. Compared to the suspension having a dry particle weight of 3 mg/ml at the time the particles having a diameter of 16.8±6.7 µm was administered in the first embodiment, the dry particle weight was approximately 0.2 mg/ ml at a dilution of 1/16. Likewise, the particles having a diameter of 4.5±1.7 µm was suspended in Williams' E culture medium containing 10% fetal bovine serum. The particles are small and cannot be measured by a cell counter, thus, the suspension having the same concentration as the particles having a diameter of 16.8±6.7 µm was manufactured at the dry particle weight.

Three hours after cell sowing, the particles were administered to the cell by replacing with the Williams' E culture medium containing 10% fetal bovine serum in which the particles were suspended. 24 hours after cells sowing, the serum-free Williams' E culture medium was replaced, 48 hours after cell sowing, the Matrigel (BD) was replaced, and subsequently, the serum-free Williams' E culture medium was replaced every 24 hours and the culturing was continued for five days

The cells cultured for five days on a nanopillar culture sheet were pre-fixed in 0.1 M phosphoric acid buffer (pH 7.4) containing 2.5% glutaraldehyde, and were post-fixed in phosphoric acid buffer containing 1% osmium tetroxide. After the nanopillar sheet to which the cells were adhered was embedded in a 1.5% concentration of low melting point agar (Sea plaquer agarose, Lonza) and subjecting to a dehydration treatment with an ethanol dilution series, polystyrene was dissolved in propylene oxide. An Epon-Alardite mixed resin was penetrated into the agar in which the cells were embedded and polymerized at room temperature. A semi-thin section having a thickness of 400 nm was prepared from the resin embedded sample using ultramicrotome. The fragment was adhered to a glass slide, stained with 1% Toluidine Blue, and observed with an upright optical microscope.

If the cross section of the aggregate is observed at the aforementioned semi-thin section, the cell aggregate to which the particles having a diameter of 16.8±6.7 µm were administered can encompass numerous particles on the inside of the aggregate, and this part is exposed on the outside of the aggregate, thus, it was considered that the culture medium component was supplied to the inside of the aggregate via the particles. However, in the case of particles having a diameter of 4.5±1.7 µm, the particles were encompassed on the inside of the cell aggregate, and the surface was not determined to be in contact with the culture medium. From this result, it has been suggested that the efficiency at which the culture medium components permeate into the aggregate the inner cell is different depending on the size of the particles.

Note that, an explanation has been omitted regarding the isolation and culturing of the rat hepatocytes, the method for production of the gelatin particles already explained in the first embodiment.

### Third Embodiment

The third embodiment explains the example showing that the effect of the metabolic enzyme cytochrome P450 (CYP3A) activity maintenance of rat hepatocytes is difference not only due to the diameter of the particles but due to the amount of the particle administered.

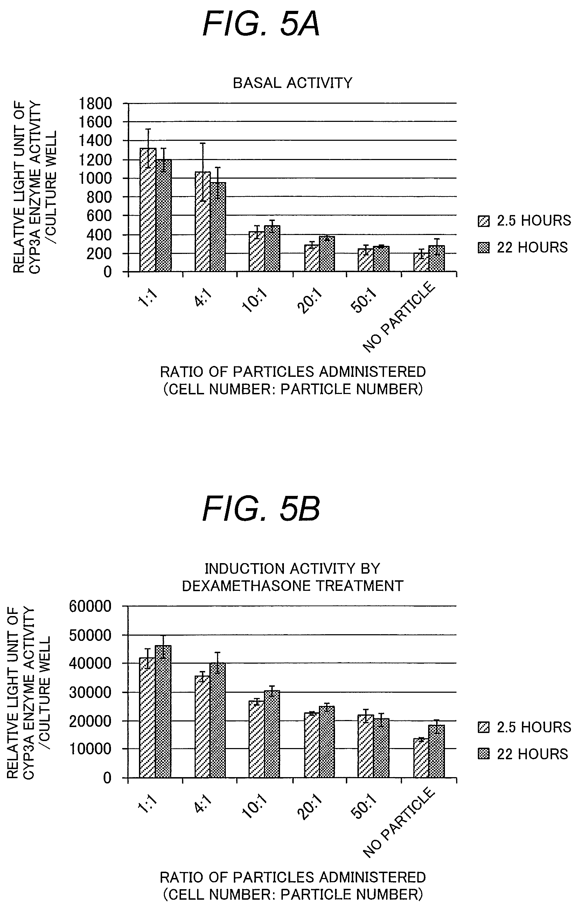

Fig. 5A and Fig. 5B are drawings illustrating examples in which the basal activity of CYP3A and the drug-induced activity were measured in the embodiments, Fig. 5A is a drawing showing the ratio at which the particles were administered relative to the cell number and the example in which the duration to which the particles were administered was changed so that the rat hepatocytes were cultured for 10 days, and the basal activity of metabolic enzyme cytochrome P450 (molecular species CYP3A) was calculated, and Fig. 5B is a drawing showing the ratio at which the particles were administered relative to the cell number and the example in which the duration to which the particles were administered were changed so that the rat hepatocytes were cultured for 10 days, treated with the induction drug dexamethasone, and the induction activity of metabolic enzyme cytochrome P450(molecular species CYP3A) was measured.

In the embodiment, a 96-well nanopillar culturing equipment material was used and the rat hepatocytes were sown in the same manner as the first embodiment. The particles having a diameter of 16.8±6.7 µm were swollen with Williams' E culture medium containing 10% fetal bovine serum, and 2.5 hours or after 22 hours after cell sowing, the particle suspension was administered onto the cells, so that cell number:particle number was 1:1, 4:1, 10:1, 20:1, 50:1. 24 hours after cell sowing, the particles were administered to the cultured cell by replacing the culture medium in which the cells were cultured from Williams' E culture medium containing 10% fetal bovine serum to the serum-free Williams' E culture medium in which the particles were suspended. 48 hours after cell sowing, the Matrigel (BD) was replaced, and subsequently, the serum-free Williams' E culture medium was replaced every 24 hours and the culturing was continued for 10 days.

When the culture medium was replaced on days 8 and 9 of culturing, the hepatocytes were exposed to dexamethasone for 48 hours using serum-free Williams' E culture medium containing dexamethasone (50 µm) for inducing the expression of CYP3A in cells of the enzyme induction testing section by the drug. Serum-free Williams' E culture medium was used in cells of the basal activity testing section of CYP3A.

48 hours after the start of the dexamethasone treatment, the activity of metabolic enzyme cytochrome P450 (molecular species CYP3A) of hepatocytes was measured using P450-Glo™ Assay (Luciferin-IPA, Promega).

The 4-well mean value and the standard deviation were determined for each testing section, and are shown in Fig. 5A and Fig. 5B. As is clear in the drawings, a higher CYP3A activity than the aggregate without the particles was clearly detected in both of the basal activity testing section and the induction activity testing section for cells in which the particles were administered at a cell number:particle number greater than 20:1 with respect to the cell number in the unit culturing area. Furthermore, a higher activity was detected by increasing the ratio of the particles. Specifically, regarding the basal activity, a high activity was detected when the cell number:particle number was at least 4:1. Regarding the induction activity, the effect of the particles was recognized even when if the cell number:particle number was 50:1. From this result, if the cell number:particle number is higher than 50:1, the effect of the particle is recognized, but it is desirable that the cell number:particle number is at least 4:1.

The aforementioned result, in addition to maintaining the activity of cells in which the supply of nutrients and oxygen from the culture medium improved by the particles between cells is considered to be the reason that the exposure of cells to the drug dexamethasone administered to the culture medium increases to make enzyme induction stronger, and the detection efficiency of enzyme activity improves by the increasing the permeability into cells of Luciferin-IPA which is a detection reagent.

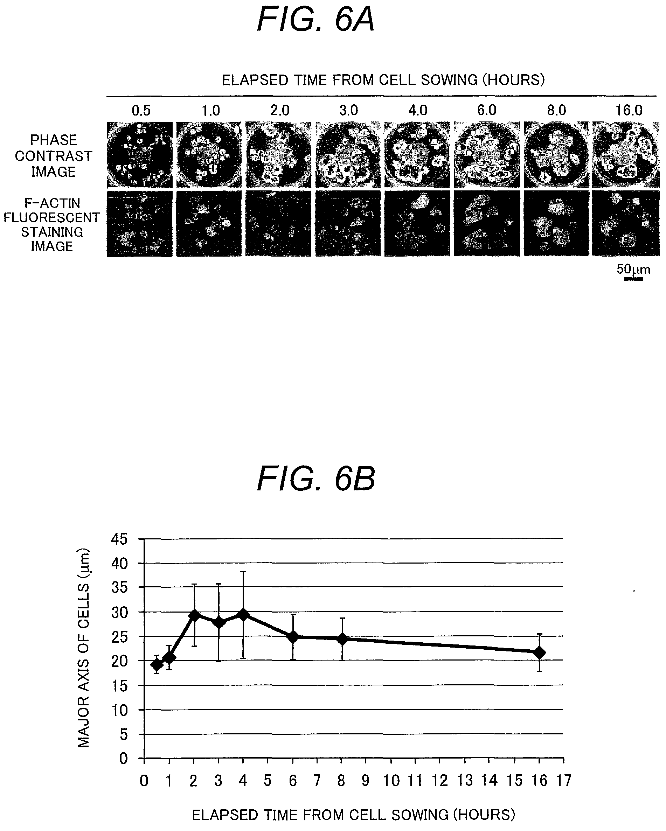

Fig. 6A is an example showing the transformation (change over time) of the cells according to the elapsed time after the rat hepatocytes were sown in the low-adhesion culturing vessel in the embodiments by a phase-contrast microscope and the distribution of F-actin, and Fig. 6A are microscope photographs which used an inverted microscope to observe cell formation over time after cell sowing, the upper level are phase contrast microscopic images, and the lower level are fluorescence microscope images which stained F-actin with rhodamine-labeled phalloidin. The phase contrast images show the entire structure, and the outline of the individual cells is shown by the F-actin fluorescent staining images. However, Fig. 6B is an example showing the change over time in which the major axis of an arbitrarily chosen 60 cells was measured after the cell sowing in a microscope photograph and the mean value and the standard deviation of the major axis were determined.

The procedure of the rhodamine-labeled phalloidin staining of Fig. 6A is as follows. Cells fixed with 4% paraformaldehyde were washed with 0.1 M phosphoric acid buffer (pH 7.4). Non-specific staining was prevented, and thus, after treating the cells with a Protein Block (DAKO) for 30 minutes and treating with a rhodamine-labeled phalloidin (Invitrogen) diluted to 1/40 with PBS for 30 minutes, the cells were washed with PBS containing 0.075 % Brij 35 (Sigma-Aldrich).

The cells were isolated 0.5 hours after being sown, and the shape of the cells was round and the average diameter was no more than 20 µm. After 1.0 hour, the two-dimensional structures of the cells shown in the photographs became somewhat large, and the average diameter slightly exceeded 20 µm.

After sowing for 2.0 hours, the cells adhered to the culturing surface, the average diameter of the cells shown in the photograph approached 30 µm, and the variation of the major axis of the cells became large. The image of F-actin shows that the cells adhere to the equipment material to become three-dimensionally thin. These cells were determined to have the same formation as cells sown for 3.0 hours and 4.0 hours, and furthermore, the variation of the major axis of the cells became large. At 4.0 hours, the intercellular adhesion was determined by the intracellular interaction.

Sowing for 6.0 hours formed intercellular adhesion between numerous cells, and the image in which the cells adhere to the equipment material to become three-dimensionally thin decreased. The average of the major axis of a cell was in the range of 25 µm, and the variation was reduced thereby. The same tendency was observed at 8.0 hours and 16.0 hours.

In the embodiment, as shown in Fig. 5A and Fig. 5B, a remarkable difference in CYP3A activity could not be observed in cells in which the particles were administered 2.5 hours after sowing and cells in which the particles were administered 22 hours after sowing. Therefore, in the present invention, it is understood that if the sown cells settle on the culturing surface of the culturing equipment material, the effect of cellular activity maintenance due to the particle can be obtained by administering the particle onto the cells.

Note that, an explanation has been omitted regarding the isolation and culturing of the rat hepatocytes, the method for production of the gelatin particles, and the CYP3A activity measurement procedure already explained in the first embodiment.

### Fourth Embodiment

The embodiment shows that the effect of the activity maintenance of rat hepatocytes is different depending on the amount of the particle administered.

When the activity of mitochondria dehydrogenase of hepatocytes which were cultured for five days was measured by the WST-1 assay, the example in which the measurement value increased is explained by the increase of the amount of particle administered to the cells. The WST-1 test is a method for measuring the activity of mitochondrial respiratory enzymes (succinate-tetrazolium reductase) of live cells, and if the measurement value is high, it indicates that the respiratory activity of the spheroid is high.

The rat hepatocytes were sown using a 96-well nanopillar culturing equipment material in the same manner as in the first embodiment. The particle having a diameter of 16.8±6.7 µm was swelled with Williams' E culture medium containing 10% fetal bovine serum, and 2.5 hours after cell sowing, or after 22 hours, the particle suspension was administered onto cells so that the cell number:particle number was 1:4, 1:2, 1:1, 2:1, 4:1, 8:1, 10:1, 20:1, 40:1, 50:1, and 80:1. 24 hours after cell sowing, the serum-free Williams' E culture medium was replaced, and 48 hours after cell sowing, Matrigel (BD) was replaced with serum-free Williams' E culture medium containing 0.25 mg/ml total protein, and subsequently, the serum-free Williams' E culture medium was replaced every 24 hours and cultured for five days.

The activity measurement used Premix WST-1 (Takara Bio Inc.). After culturing the hepatocytes for five days, the culture medium from each well of the 96-well plate into which the cells were inserted was completely removed, and washed twice with Hank's solution. The washing fluid was removed and 65 µl of Hank's solution to which 10% (v/v) concentration of WST-1 was added was inserted, and reacted in an incubator maintained at 37 degrees and a 5% carbon dioxide gas concentration for one hour. After the reaction, the 96-well was set in a plate reader, and the absorbance of the two wavelengths (440 nm and 650 nm) was measured at the half-value width of 5 nm. The value was determined by subtracting the value of 650 nm which is a control wavelength from the value of 440 nm.

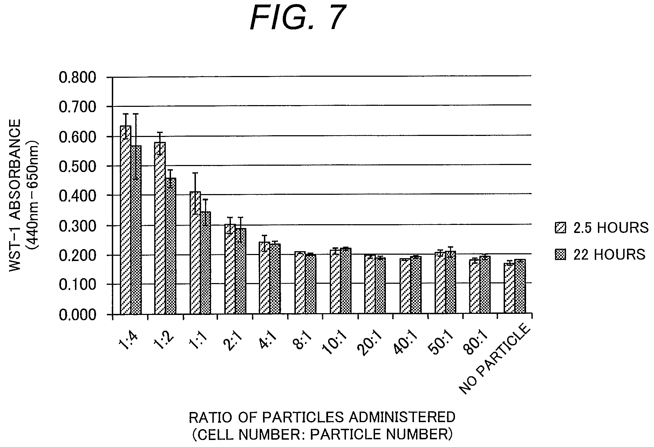

Fig. 7 indicates the ratio at which the particles were administered relative to the cell number, and the effect when the duration to which the particles were administered was changed so that the rat hepatocytes were cultured for five days, the activity of mitochondria dehydrogenase was measured by WST-1 assay, and the 3-well mean value and the standard deviation was determined for each testing section. Regarding the results of the measurement, as is clear from Fig. 7, the effect of the particles was recognized when the cell number:particle number was 1:80, i.e., the number of particles was more than 1/80 relative to the number of cells, but the measurement value of WST-1 remarkably increased by administering particles in excess of 4:1, and furthermore, the value further increased by increasing the amount administered to 1:1. The aforementioned result, in addition to maintaining the activity of cells in which the supply of nutrients and oxygen from the culture medium improved by the particles between cells, is considered to be the reason that the detection efficiency of the respiratory activity improves by increasing the permeability of WST-1 which is a detection reagent into cells.

Note that, an explanation has been omitted regarding the isolation and culturing of the rat hepatocytes, the method for production of the gelatin particles, and the procedure by which the particle is swollen in the culture medium which were already explained in the first embodiment.

### Fifth Embodiment

The embodiment shows an example of the process of the cell aggregate formation when the particles are administered in different amounts and the cell aggregate formation which is formed. Furthermore, the example indicates that the efficiency at which the bile canaliculus which is a hepatic tissue specific structure is formed is different depending on the amount of particle administered.

The development of a cell testing system for measuring the ratio excreted to the biliary ducts among the drugs taken up in the hepatocytes is desirable in pharmacokinetics testing in research for developing new drugs. To achieve this, a culturing system in which a bile canaliculus can be efficiently formed, and which easily verifies the excretion capacity is provided.

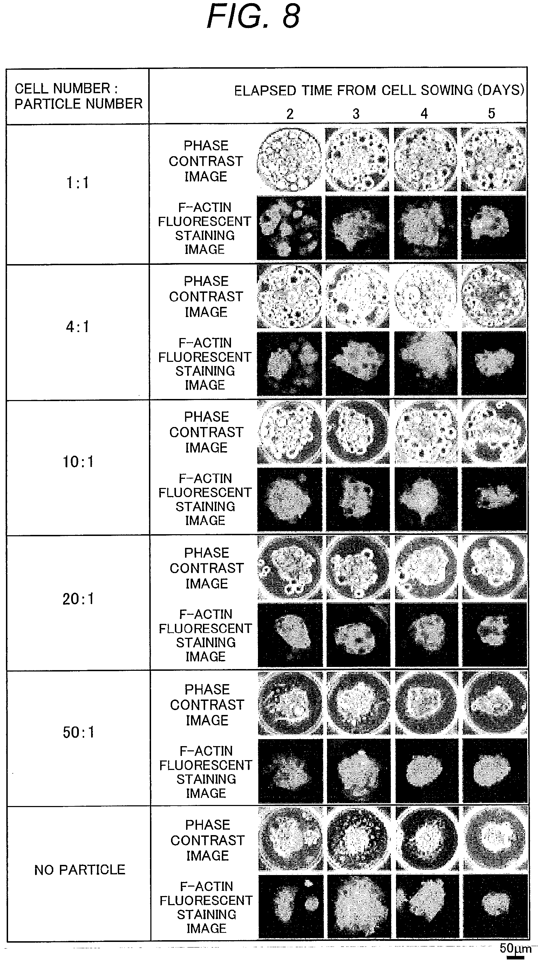

Fig. 8 is a drawing illustrating an example of the change of the cell aggregate formation over time when particles were administered at different ratios relative to the cell number and cultured for five days. The phase contrast images show the entire structure of the particles and the cells, and the F-actin fluorescent staining image is a drawing showing the distribution of cells. Namely, the process of the cell aggregate formation when cultured for five days by administering the particle having a diameter of 16.8±6.7 µm to the rat hepatocytes so that the cell number:particle number was 1:1, 4:1, 10:1, 20:1, 50:1 is shown. The upper level of each column is the phase contrast microscopic images and the lower level is the fluorescence microscope images which stained the F-actin with rhodamine-labeled phalloidin.

It was understood that when the particles were administered, the cell aggregate formed when cultured for three days. When the particles were not administered, the cells which were cultured for three days adhered to the culturing vessel, thus, it was shown that the cell aggregate quickly formed in the range of one day by administering the particle.

In hepatic tissues in vivo, F-actin forms the backing structure of a membrane, and the cells have a polygonal three-dimensional structure. It is possible to verify whether or not the tissues are similar to cells in vivo by observing the distribution of F-actin. Until now, regarding the cell aggregate formed in a nanopillar culturing vessel, F-actin formed the backing structure of a membrane similar to that in vivo (refer to WO2010/079602).

In the embodiment, it was considered that in the cases of the aggregate formed when the cell number:particle number was 50:1, or when there was no particle, F-actin formed a backing structure of a membrane similar to that in vivo. In comparison thereto, when the particles were administered at a cell number:particle number of 20:1, 10:1, 4:1, or 1:1, an image showing a strong linear or spot-like fluorescence was seen on the cell surface to which the gelatin particles adhere, and the possibility of an adhesion point or a stress fiber was considered. These are the structures formed when the cells adhere to the extracellular matrix. Therefore, it was considered that the cells formed an adhesion structure on the gelatin particle surface in the same manner as the adhesion with the extracellular matrix.

From this result, it was considered that the cell number:particle number was preferably no more than 50:1 to form a backing structure of a membrane similar to in vivo.

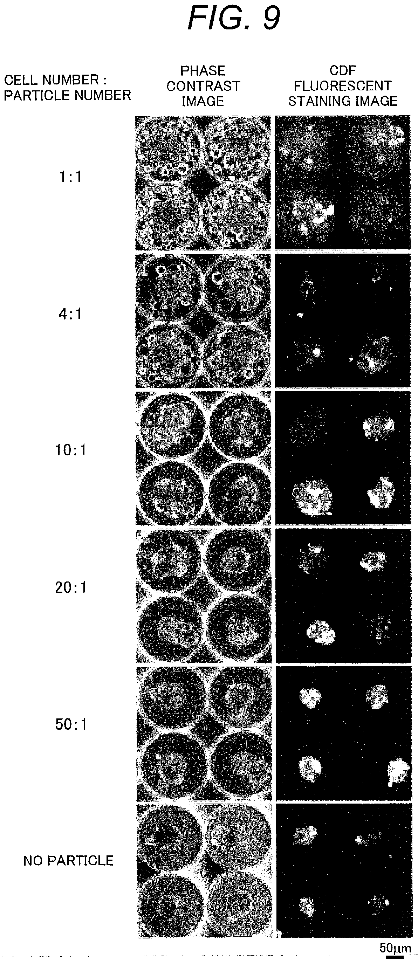

Fig. 9 is a drawing illustrating an example of the uptake of the fluorescent reagent when the particles were administered at different ratios relative to the cell number and cultured for five days and the excretion to the bile canaliculus. Specifically, Fig. 9 is an example showing the excretion capacity of the bile canaliculus formed on the intercellular adhesion surface of the hepatocytes using fluorescent-labeled reagents carboxydichlorofluorescein diacetate (CDFDA, Molecular Probe) cultured for five days. CDFDA administered in the culture medium is taken up into the cells and metabolized into carboxydichlorofluorescein (CDF), and is excreted in the bile canaliculus via the transporter multidrug resistance-associated protein 2(MRP2) of the cell membrane facing the bile canaliculus. The higher the biliary excretion function of the cultured cells, the more drugs are excreted into the bile canaliculus to remain between cells, thus, a strong spot-like or linear fluorescence was observed under a fluorescence microscope.

The procedures of the CDFDA treatment of the embodiments are as follows. The culture medium of the hepatocytes which were cultured in a 24-well nanopillar culture plate was removed and washed with Krebs-Henseleit buffer (K-H buffer) twice. 400 µl of the K-H buffer containing 5 µm of CDFDA was inserted in each well, and after being placed in a 37 degrees incubator for 20 minutes, washed with K-H buffer twice, and observed with an inverted type fluorescence microscope.

As the result of the observation, when the cell number:particle number was 50:1, 20:1, or 10:1, a strong spot-like or linear fluorescence which is considered to be the CDF excreted in the bile canaliculus was recognized. However, at a cell number:particle number of 4:1, the cell aggregate was amorphous and flat, and a linear fluorescent image was recognized, thus, it was considered that the bile canaliculus was spread flat.

With respect thereto, when the cell number:particle number is 1:1, if the auto-fluorescence of the particle is excluded, the fluorescence has been observed to be granular in the cells, thus, it was observed that the CDF was not excreted in the bile canaliculus but taken up and stored within the cells.

The bile canaliculus is formed on the intercellular adhesion surface, and thus, is necessary for intracellular interaction. Further, it is known that the proteins present on the inner side of the cellular membrane of the intercellular adhesion surface play an important role in the expression of transporter MRP2 for excreting drugs from within cells to the bile canaliculus. Therefore, it was considered that the fact that it was difficult to regenerate a bile canaliculus when there were many particles was barely regenerated was the cause that particles present in excess between cells inhibited the adhesion between cells.

As shown in Fig. 8, to form F-actin cellular membrane lining structures by intercellular adhesion, it was preferable that the cell number:particle number was no more than 50:1, but to detect excretion to the bile canaliculus, it is desirable that the cell number:particle number is from 50:1 to 4:1, and more desirably from 50:1 to 10:1. It was considered that this was reason that it was necessary to not only promote intercellular adhesion to improve both the uptake of the pharmaceutical agent and the excretion, but to form such that the surface in contact with the culture medium via the particle.

The fourth embodiment indicates the possibility that the supply of culture medium components to the inside of the cell aggregate is improved by encompassing numerous particles, but on the one hand, it was not clear in the embodiment that the excretory functions specific to hepatic tissues decreased due to the presence of the excess particles. There are contradictory conditions in particle administration, but it is desirable to determine a suitable amount of particles in accordance with the use purpose of the cell aggregate.

Note that, an explanation has been omitted regarding the isolation and culturing of the rat hepatocytes, the +method for production of the gelatin particles, and the procedure by which the particle is swollen in the culture medium which were already explained in the first embodiment, and the procedure of rhodamine-labeled phalloidin staining shown in the third embodiment.

### Sixth Embodiment

The embodiment explains one example of the culturing vessel provided with a cell adhesion region for capturing cell aggregate by forming a plurality of projections in the region of the culturing surface of a low cell-adhesive material which is used in each of the aforementioned embodiments.

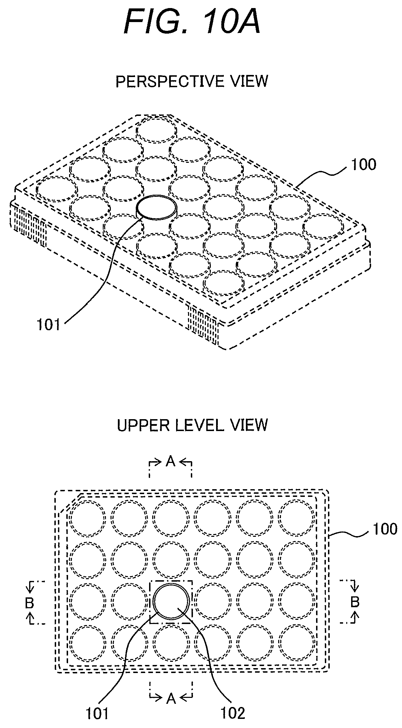

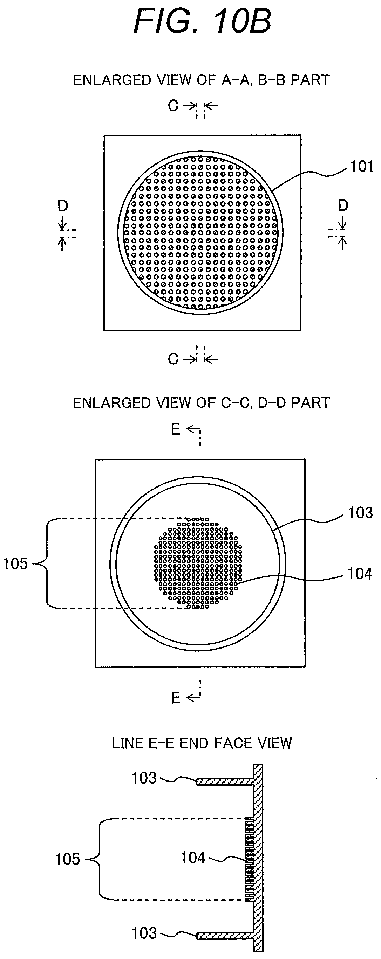

In Fig. 10A, the upper level and the lower level indicate the perspectives of a multi-well plate 100 for a cell culture and the upper surface. The culturing surface of a bottom surface 102 of a well 101 shown in A-A and B-B is subjected to fine three-dimensional processing. In Fig. 10B, the upper level, the middle level, and the lower level show the expansion of the A-A and B-B portions, the expansion of the C-C and D-D portion, and the line E-E end face of Fig. 10A. The culturing surface subjected to fine three-dimensional processing is a hole 103 shown in the expansion of the C-C and D-D portions and the line E-E end face, and thus, is a drawing illustrating an example in which an aggregation region 105 of a projection 104 is molded in the center of the hole 103.

Fig. 11 is an example of a scanning electron microscope photograph of the culturing surface on which the aggregation region 105 of the projection 104 molded in the center of the hole 103 is molded.

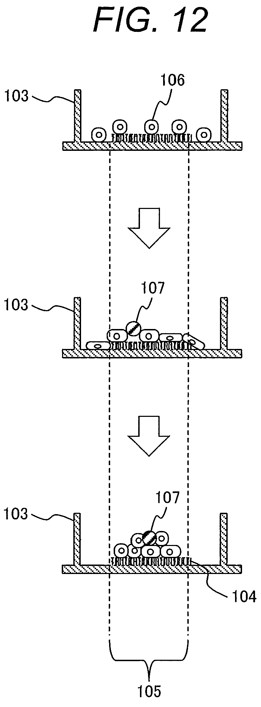

Fig. 12 shows an example of a schematic illustration showing that the cell aggregate encompassing the particle adheres to the aggregation region 105 of the projection 104 molded in the center of the hole 103 by sowing cells 106 to the hole 103 of the culturing surface shown in Fig. 10B and Fig. 11, and administering gelatin particles 107 at the time the cells 106 are adhered to the bottom surface.

Note that the formation of the local cell adhesion region for capturing cells on the culturing surface is not limited to the projection of the embodiments, and may be formed by arbitrarily patterning cell adhesion substances such as extracellular matrix components such as collagen and laminin, antibodies bound to the cell membrane surface, sugar chains bound to the cell membrane surface, poly-L-lysine, etc.

The culturing vessel of the aforementioned example can maintain the particulate-encompassing cell aggregate within the culturing vessel by forming a cell adhesion region which can capture cell aggregates on a low-adhesion culture surface. Furthermore, by providing a partition preferably on the culture surface, the cell number and the particle number which form the aggregate is controlled to the desired number, and the particulate-encompassing cell aggregate having the desired size is formed. The particulate-encompassing cell aggregate adhering to the culturing vessel in the embodiments may be used as the material of cell testing.

### Seventh Embodiment

The embodiment explains an example of a human cell testing method for encompassing a particle.

The first embodiment and third embodiment showed the effect that the cell aggregate encompassing the particle maintained the activity of the metabolic enzyme cytochrome P450 (molecular species CYP3A) for a longer period than the aggregate which did not contain the particle and the conventional single layer culturing method.

Cytochrome P450 is an enzyme which metabolizes drugs which are taken up from the blood into hepatocytes, thus, metabolism in pharmacokinetics, drug interactions by CYP enzyme induction, hepatotoxicity by CYP metabolites, etc., are the targets for research in research for developing new drugs. Specifically, there are interspecific differences in the substrate specificity and the metabolic activity of an enzyme, thus, it necessary not only to perform testing on animals, but also to perform testing on humans, and accordingly, a testing method which uses isolated human hepatocytes has been developed. It is possible to stably perform tests which use isolated human hepatocytes over a longer period of time in which activity is easily lost, by using cell aggregate for encompassing the aforementioned microscaffold particles in cell testing.

First, an example of CYP metabolite analysis will be explained. Frozen stored human hepatocytes were defrosted, a cell concentration of 5×10⁵ cells/ml was suspended in Williams' E culture medium containing 10% fetal bovine serum, and sown at a density of 1×10⁵ cells/cm² per culture area of the culturing vessel. The culture plate was placed in a 37-degree, 5% carbon dioxide gas concentration incubator and the cells were cultured. The cell aggregate encompassing the particles were formed by swelling the particle having a diameter of 16.8±6.7 µm in the culture medium, and administering the particle suspension onto the cells so that the particle number was greater than the cell number. The pharmaceutical agent metabolized by CYP was administered in the culture medium, and the cells and the culture medium are recovered after two days and after seven days. The drug metabolite in the cells or in the culture medium was identified by performing a deproteinization treatment on the cell lysate or the culture medium to analyze the recovered sample solution by mass spectrometry.

Next, an example of CYP enzyme induction testing for drugs which are the cause of drug interactions will be explained. The cell aggregate encompassing the particles were formed by culturing frozen stored human hepatocytes and administering the particles in the same manner as the example of metabolite analysis. The culture medium containing the drug to be tested was cultured from 48 hours to 72 hours, and then, a detection reagent which specifically reacts with each molecular species of CYP was used to measure the activity of the CYP enzyme. The CYP enzyme activity of cells which were not treated with the object drug was made as the basal activity, the CYP enzyme activity of cells which were treated with the object drug was made as the induction activity, and the induction rate was calculated by dividing the induction activity value by the basal activity value.

As the detection reagent of CYP enzyme activity, the CYP enzyme activity due to the drug to be tested can be measured by the quantification of the fluorescence using a P450-Glo™ Assay series (Promega), and the like, shown in, for example, the first embodiment and third embodiment. Alternatively, CYP induction activity can be measured using known substances which specifically metabolize each molecular species of CYP to quantify the metabolite by mass spectrometry.

For predictions of drug interactions, methods for measuring the range of the drug interaction due to the drug to be tested by measuring and calculating the induction rate by drugs known to strongly induce CYP, for example, rifampicin in parallel with the drug to be tested, and comparing with the induction rate the drug to be tested may be provided.

Furthermore, an example of liver toxicity analysis by a CYP metabolite will be explained. Similarly to the example of metabolite analysis, the cell aggregate encompassing the particles were formed by culturing frozen stored human hepatocytes and administering the particle. The toxicity of the drugs was evaluated by changing the concentration of the pharmacological agent to be tested and administering to the culture medium, and measuring the variation of the cell number over time. For the measurement of cell number, examples such as the WST-1 method shown in the fourth embodiment or the ATP quantification method can be provided. A plurality of testing examples are provided as the explanation of the seventh embodiment, but it is not limited thereto.

As previously stated, the cell culturing method according to the present invention is a culturing method characterized by adhering cells to the culturing surface of the culturing vessel, wherein cell testing performed with the conventional single layer culturing method may be used in the same procedure.

Furthermore, the single particulate culture and the particulate-encompassing cell aggregate according to the present invention are three-dimensional cell aggregates having structures more similar to the in vivo structure than to the single layer culture, and cell function is maintained by supplying nutrients and oxygen within the culture medium via the particles, and in cell testing, the drugs administered in the culture medium permeate via the particle, thus, a testing system having a high reproducibility which stably detects the drug response of cells over a long period of time can be provided.

### Reference Signs List

100... multi-well plate for cell culture
101... well
102... culturing surface
103... hole formed in culturing surface
104... projection molded in the hole bottom surface center
105...aggregation region of the projection molded in the hole bottom surface center
106... hepatocytes
107... gelatin particles

## Claims

1. A cell culturing method for forming a cell aggregate for encompassing a particle by sowing cells in a culturing vessel and administering particles that are cell-adhesive and substance-permeable.

2. The cell culturing method according to claim 1, wherein the particle size of the particle to be encompassed in the cell aggregate is in the range of 1/10 to 10 times the cell.

3. The cell culturing method according to claim 1, wherein the particle size of the particle to be encompassed in the cell aggregate is at least 1/5 of the cell.

4. The cell culturing method according to claim 1, wherein when administering the particle to cells, the particle number is more than 1/80 with respect to the number of cells per unit culturing area.

5. The cell culturing method according to claim 1, wherein when administering the particle to cells, the particle number administered is more than 1/50 with respect to the number of cells per unit culturing area.

6. The cell culturing method according to claim 1, wherein the cell aggregate is maintained in the culturing vessel by forming at least one cell adhesion region which can capture the cell aggregate in a low-adhesion culturing vessel.

7. The cell culturing method according to claim 1, wherein a partition is provided on the culture surface on which the cells adhere such that the particulate-encompassing cell aggregate having the desired size which controls the number of cells which produce the cell aggregate within the partition and the number of particles administered onto the cells which settled in the partition, and which encompass the desired size of particles is formed.

8. The cell culturing method according to claim 1, wherein the cells are isolated hepatocytes, hepatocytes differentiated and induced from stem cells, hepatic precursor cells, or hepatoma-derived cells.

9. A particulate culture carrier comprising particles that are cell-adhesive and substance-permeable which are encompassed in a cell aggregate produced by administering the particles onto the sown cells in the culturing vessel.

10. The particulate culture carrier according to claim 9, wherein the particle size of the particle to be encompassed in the cell aggregate is in the range of 1/10 to 10 times the cell.

11. The particulate culture carrier according to claim 9, wherein the particle size of the particle to be encompassed in the cell aggregate is at least 1/5 of the cell.

12. The particulate culture carrier according to claim 9, wherein the cells are isolated hepatocytes, hepatocytes differentiated and induced from stem cells, hepatic precursor cells, or hepatoma-derived cells.

13. A particulate-encompassing cell aggregate formed by sowing cells in a culturing vessel and administering particles that are cell-adhesive and substance-permeable onto the cells.

14. The particulate-encompassing cell aggregate according to claim 13, wherein when administering the particle to cells, more than 1/50 is administered with respect to the number of cells per unit culturing area.

15. The particulate-encompassing cell aggregate according to claim 13, wherein the cells are isolated hepatocytes, hepatocytes differentiated and induced from stem cells, hepatic precursor cells, or hepatoma-derived cells.
